# EUROPEAN PATENT APPLICATION

(11) **EP 2 491 862 A1**
(43) Date of publication of application: **29.08.2012**
(21) Application number: 12156218.5
(22) Date of filing: 20.02.2012
(51) Int. Cl.: A61B 6/00, A61B 6/02

(54) **Apparatus for tomosynthesis and mammography**

(30) Priority: 25.02.2011 IT BO20110084
(71) Applicant: I.M.S. Internazionale Medico Scientifica S.r.l., 40044 Pontecchio Marconi (Bologna) (IT)
(72) Inventor: Albanese, Achille, 40044 PONTECCHIO MARCONI (Bologna) (IT); Toniolo, Bruno, 40044 PONTECCHIO MARCONI (Bologna) (IT); Vecchio, Sara, 40044 PONTECCHIO MARCONI (Bologna) (IT); Vignoli, Paolo, 40044 PONTECCHIO MARCONI (Bologna) (IT); Borasi, Giovanni, 40044 PONTECCHIO MARCONI (Bologna) (IT)
(74) Representative: Bianciardi, Ezio

(57) **Abstract**

An apparatus (1) for performing tomosynthesis and mammography of a breast (M) of a patient, comprises: an X-ray detector device (2), designed to receive and detect X-rays in a first, detection plane (3); a plurality of X-ray sources (4), stably positioned in a second plane (5) which is substantially at a right angle to the first, detection plane (3) in a plurality of predetermined positions (P1, P2, P3, P4, P5) relative to the detector device (2), each of the sources (4) being individually activatable for emitting a corresponding X-ray beam (F) towards the first, detection plane (3); a region (R2) for positioning the breast (M); a processing and control unit (6), connected to the X-ray sources (4) for activating them individually and connected to the detector device (2) for receiving a signal (s1) relating to the X-rays which passed through the breast (M) and were detected by the detector device (2), the unit (6) being designed to derive from the signal (s1) at least one radiographic image representative of the internal structure of the patient's breast (M).

## Description

This invention relates to an apparatus for performing tomosynthesis (in particular, digital breast tomosynthesis) and mammography.

Known in the prior art are apparatuses designed to perform both digital breast tomosynthesis (DBT) and mammography.

These apparatuses comprise a source configured to emit X-rays and a detector configured to receive the X-rays emitted by the source.

It should be noted that the patient's breast being analysed is interposed between the X-ray source and the detector in such a way that the X-rays pass through it.

Generally speaking, the breast is placed on a suitable rest and compressed by a plate known as compressor.

As is known, in the breast being analysed X-rays are absorbed to a different extent by parts affected by tumour growths or the like as compared to parts without tumour growths. A radiographic image obtained from the X-rays received by the detector can thus be analysed to identify suspected tumours.

To use the apparatus for performing mammography, the X-ray source, the detector and the patient's breast must be positioned in a predetermined space relationship with each other. Processing of the X-rays received by the detector makes it possible to obtain a two-dimensional image of the patient's breast.

To perform digital breast tomosynthesis, on the other hand, the X-ray source is movable to allow the X-rays to be generated from a plurality of angles relative to the patient's breast.

One advantage of these apparatuses is the possibility of combining tomosynthesis with mammography in order to obtain a very effective and accurate diagnosis based on the combination of both techniques.

A need which is strongly felt by operators in this field is that for an apparatus for performing both tomosynthesis and mammography at a high speed while at the same time guaranteeing high quality X-ray images during both mammography and tomosynthesis. This invention therefore has for an aim to satisfy the aforementioned need by providing an apparatus for performing mammography and tomosynthesis which allows mammography / tomosynthesis to be performed at high speed while at the same time allowing high-quality images to be obtained.

Another aim of the invention is to provide an apparatus for performing mammography and tomosynthesis which makes it possible to locate quickly and accurately the trajectory of a needle through the patient's body in order to take a biopsy of biological tissue from a given part of the patient's body.

Yet another aim of the invention is to provide an apparatus for performing mammography and tomosynthesis in which the diffuse or secondary radiation received by the detector is reduced.

In accordance with the invention, this aim is achieved by an apparatus for performing mammography and tomosynthesis comprising the technical features set out in one or more of the appended claims.

The technical features of the invention according to the above mentioned aims are clearly described in the claims below and its advantages are more apparent from the detailed description which follows, with reference to the accompanying drawings which illustrate a non-limiting example embodiment of the invention and in which:
- Figure 1 is a schematic front view of a preferred embodiment of the apparatus according to this invention;
- Figure 1A illustrates another view of the apparatus according to the invention where some parts have been cut away in order to better illustrate others;
- Figure 2 is a schematic front view of the apparatus of Figure 1 in another configuration;
- Figure 3 shows a detail of a variant of the apparatus of Figures 1 and 2;
- Figure 4 is a schematic side view of a detail of the apparatus of Figures 1 and 2 according to the viewing direction J1;
- Figure 5 shows a graph representing the flow of energy per unit area of an X-ray tube at a defined distance therefrom as a function of the angle to the central direction of emission in a plane at a right angle to the anode-cathode direction;
- Figure 6 shows a schematic enlarged view of a region R2 of intersection of the principal axes of emission of the X-rays from the sources of the apparatus of Figures 1-5.

With reference to the accompanying drawings, the numeral 1 denotes an apparatus for tomosynthesis and mammography according to this invention.

More specifically, it should be noted that the apparatus 1 according to the invention allows both mammography and tomosynthesis to be performed on a breast M of a patient.

Hereinafter, the term "patient" is used to mean a person, whether male or female, subjected to analysis using the apparatus 1.

The apparatus 1 comprises an X-ray detector device 2 designed to receive and detect X-rays in a first, detection plane 3 (hereinafter also referred to as detection plane 3).

It should be noted that the detector device 2 is a substantially two-dimensional detector.

The detector device 2 comprises a sensor designed to detect a flow of X-rays incident upon the detection plane 3.

It should be noted that preferably, but without limiting the invention, the sensor of the detector device 2 is a solid state sensor based on amorphous selenium.

The apparatus 1 comprises a plurality of X-ray sources 4.

Preferably, the X-ray sources 4 are X-ray tubes.

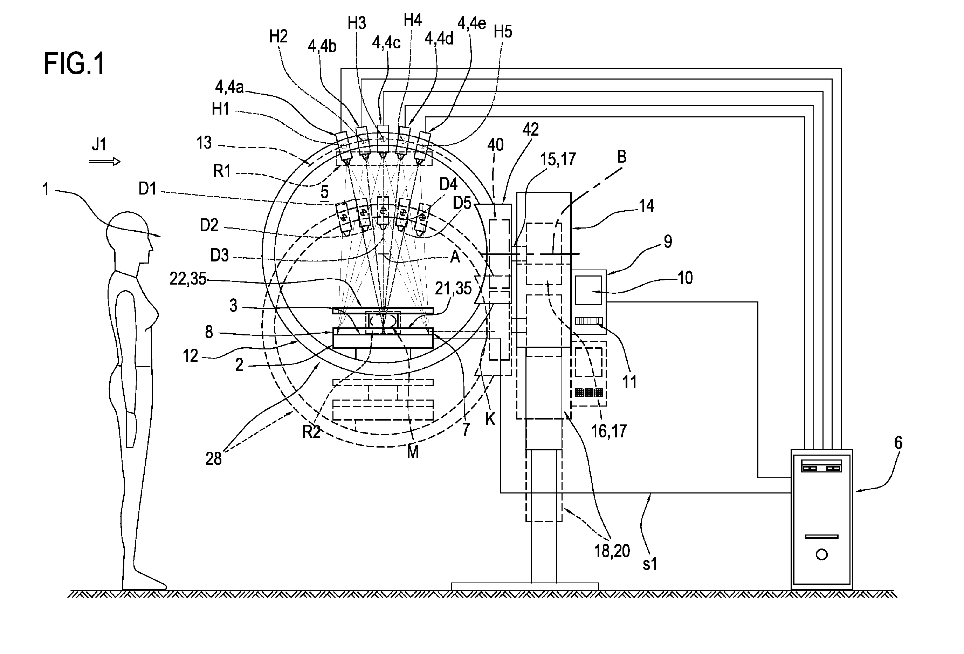

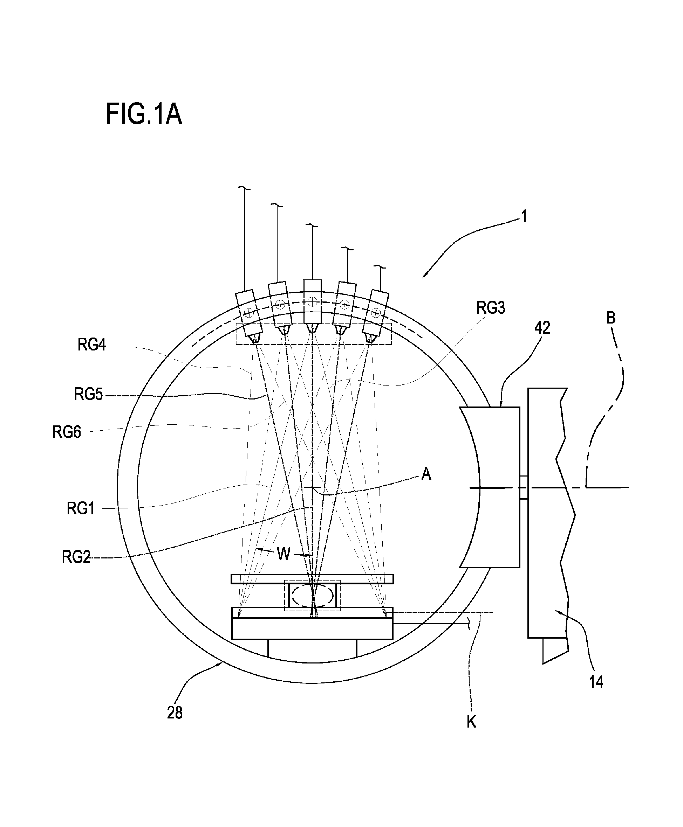

It should be noted that the term "X-ray tube" or just "tube" will hereinafter be used. This term must not however be understood as limiting the scope of the invention since the X-ray tube may, in variants of the apparatus 1 not illustrated, be substituted for X-ray sources of other kinds. Preferably, but without limiting the invention, in the embodiment illustrated in Figure 1, the apparatus 1 comprises five X-ray tubes, which are individually labelled 4a, 4b, 4c, 4d, 4e.

Each X-ray tube (4a, 4b, 4c, 4d, 4e) is positioned in such a way as to emit a corresponding X-ray beam F towards the first, detection plane 3.

The X-ray beam F is preferably a beam of slightly diverging X-rays (that is, it is conical) directed principally along a corresponding principal direction of emission (D1, D2, D3, D4, D5).

It should be noted that the principal direction of emission or of maximum emission (D1, D2, D3, D4, D5) of each tube 4 defines a central axis of emission of the respective tube 4.

As may be observed in Figure 1, each tube emits a beam of X-rays according to an emission cone (whose emission angle is adjustable).

In light of this, it should be noted that each X-ray tube (4a, 4b, 4c, 4d, 4e) is preferably equipped with a beam collimating device which is configured to adjust the divergence of the beam relative to the principal direction of emission (D1, D2, D3, D4, D5), that is, to adjust the angle of emission.

The emission focal points of the X-ray tubes (4a, 4b, 4c, 4d, 4e) lie substantially in the same plane 5 at a right angle to the first, detection plane 3. It should be noted that relative to the first, detection plane 3 the tubes 4 are oriented according to a plurality of different angles, that is, the principal directions of emission (D1, D2, D3, D4, D5) differ from each other in orientation relative to the first, detection plane 3.

Each X-ray tube (4a, 4b, 4c, 4d, 4e) is activatable individually to emit a corresponding X-ray beam F towards the first, detection plane 3.

According to the invention, the apparatus 1 also comprises a processing and control unit 6.

The processing and control unit 6 is connected to the X-ray tubes 4 in order to activate them and is also connected to the detector device 2 in order to receive a signal s1 relating to the quantity of X-rays received by the detector device 2.

The processing and control unit 6 is designed to derive from the signal s1 received a radiographic image representative of the portion of the patient's body subjected to examination (that is, the breast M).

It should be noted that the radiographic image representative of the breast M subjected to mammography / tomosynthesis is obtained by suitably processing the signal s1.

Further technical and functional features of the processing and control unit 6 are described below. It should also be noted that the apparatus 1 comprises an operator interface 9 designed to display the results of mammography and/or tomosynthesis (for example, the radiographic image/images derived from the parameter/parameters correlated with the signal s1 detected) and to receive commands from the operator.

The interface 9 preferably comprises a display 10 and a keyboard 11 for entering commands or, alternatively, a display of the touch-screen type. The apparatus 1 comprises a first frame 12 for supporting the X-ray tubes (4a, 4b, 4c, 4d, 4e) and the detector device 2.

It should be noted that the detector device 2 and the X-ray tubes (4a, 4b, 4c, 4d, 4e) are stably fixed to the first frame 12. Thus, the X-ray tubes 4 are positioned in a predetermined space relationship with the detector device 2.

In the preferred embodiment, the first frame 12 has the shape of a ring. More specifically, it should be noted that in Figure 1, the centre of the ring 28 defined by the first frame 12 is labelled A.

It should also be noted that the X-ray tubes are preferably supported by an arc 13 of the selfsame ring 28 defined by the first frame 12, while the device 2 is preferably, but without limiting the invention, positioned in the internal region of the ring 28 defined by the first frame 12.

Preferably, the X-ray tubes 4 are equispaced along the circular arc 13 of the ring 28. Still more preferably, each of the X-ray tubes 4 is spaced from the one adjacent to it along the arc 13 by an angle of approximately 10 sexagesimal degrees relative to a central point of a region or zone R2 for positioning the breast M.

As regards the position of the X-ray tubes (4a, 4b, 4c, 4d, 4e), the following should be noted. Preferably, each tube (4a, 4b, 4c, 4d, 4e) is angularly rotated about a corresponding axis (H1, H2, H3, H4, H5) at a right angle to the second plane 5 in such a way as to substantially even out the irradiation among the different X-ray tubes 4 on the first detection plane 3.

It should be noted that the axis (H1, H2, H3, H4, H5) about which each tube (4a, 4b, 4c, 4d, 4e) is rotated is preferably an axis which is at a right angle to the plane T defined by the patient's chest TO when the patient is positioned relative to the apparatus in such a way as to allow mammography / tomosynthesis to be performed.

Further, each axis (H1, H2, H3, H4, H5) intersects the straight line defining the principal direction of emission (D1, D2, D3, D4, D5) of the respective tube.

Still more preferably, the axis (H1, H2, H3, H4, H5) is proximal to a focal point of emission of the rays making up the beam F from the tube.

In other words, each tube (4a, 4b, 4c, 4d, 4e) is rotated about an axis which is at a right angle to the second plane 5 in such a way as to maximize a parameter relating to the evening out or uniformity of irradiation among the tubes 4.

As is known, the energy fluency or radiation intensity of an X-ray source per unit area, measured on the detector device 2 relative to the central axis, varies inversely to the square of the distance between the X-ray source and the detector device 2.

It is also known that the relative energy fluency of a tube 4 in a surface which is arbitrarily small and located at a given distance from the tube 4 varies with the angle formed between the ray incident upon the surface itself and the central axis of emission of the selfsame tube 4 (by way of an example, this angle is labelled W in Figure 1A). More specifically, the energy fluency is at its maximum along the direction of the central axis (D1, D2, D3, D4, D5) and decreases with distance away from the central axis.

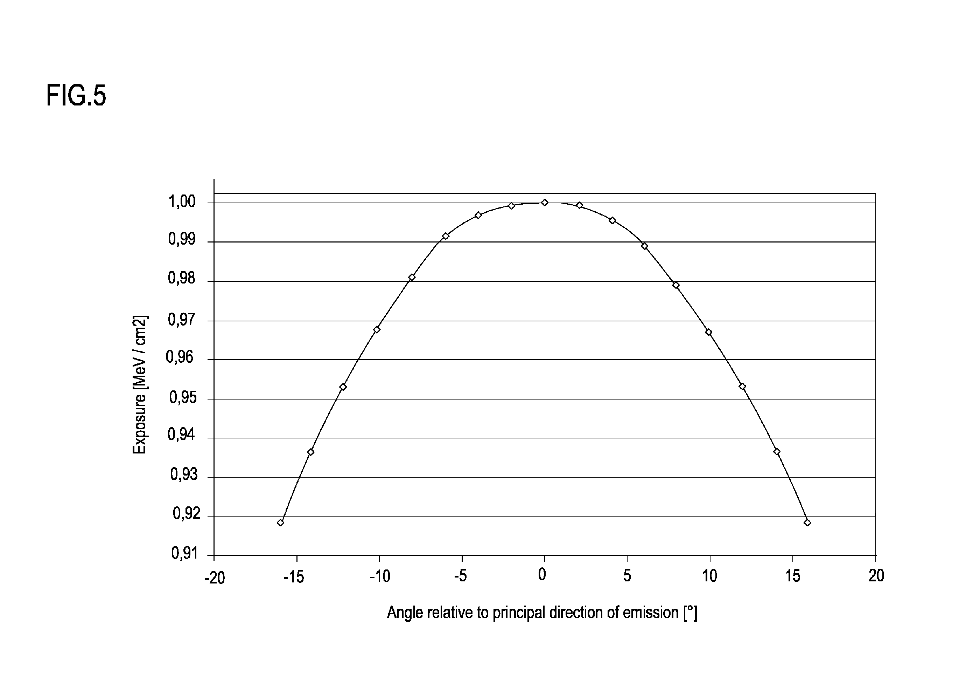

By way of an example, Figure 5 shows the energy fluency curve at a given distance from the point of emission of a tube 4 as a function of the angle formed between a ray and the central axis.

In Figure 1A, the reference labels RG1, RG2, RG3 denote three X-rays from the tube 4c and the reference labels RG4, RG5, RG6 denote three X-rays from the tube 4a.

With reference to the tube 4a and by way of an example, it should be observed that on account of rotation of the tube 4a about H1, the ray RG4 travels, from the focal point of emission, a smaller distance compared to the ray RG5 from the plane 3 to strike the detector device 2 but, on the other hand, is positioned at a larger angle to the central axis of the tube 4a (which in practice coincides with the ray RG5). Thanks to these opposite effects, it is possible to choose a direction of the central axis D1 such that in the detection plane 3, the uniformity of energy fluency is maximized between the rays RG5 and RG4 (indeed, the fact that the distance of the ray RG4 is smaller than RG5 would create a greater fluency in the plane 3 but this effect is reduced or cancelled out by the fact that RG4 is positioned at an angle greater than RG5 relative to the central axis).

In exactly the same way, the above also applies to all the other rays / tubes 4.

Thus, advantageously, the rotation of each tube 4 about the respective axis (H1, H2, H3, H4, H5) makes it possible to even out, that is, to make uniform, the energy fluency in the plane 3 of the detector device 2 (of each tube 4 and/or of the different tubes relative to one another).

It should be noted that, generally speaking, the apparatus 1 is not isocentric since the central axes of the tubes 4, which are positioned in such a way as to maximize uniformity of fluency (as described above), do not intersect at the same point.

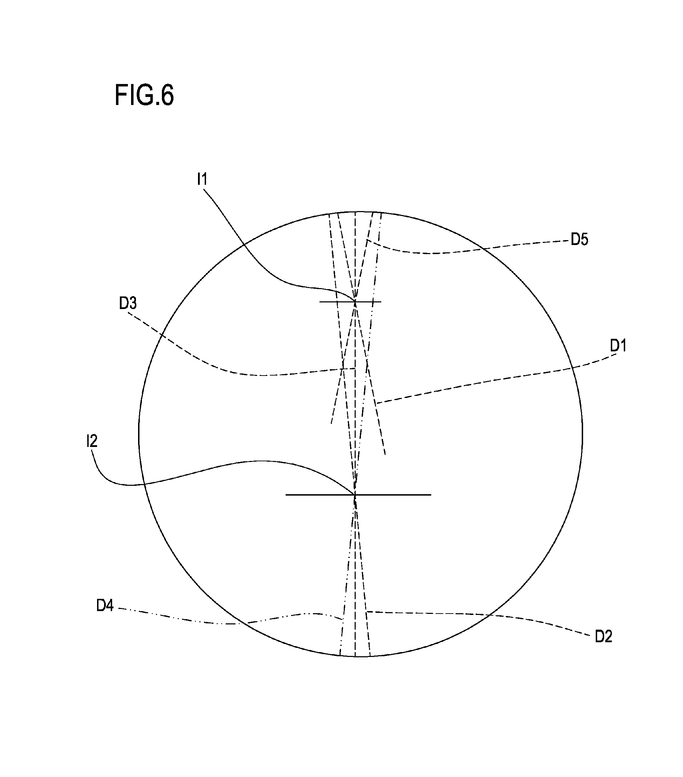

In effect, as clearly shown in Figure 6, the principal directions of emission (D1, D2, D3, D4, D5) do not intersect at the same point.

In this regard, by way of an example and without limiting the invention, Figure 6 shows the principal directions of emission D1, D3 and D5 intersecting at a first point I1 and the principal directions of emission D2, D3 and D4 intersecting at a second point I2.

Preferably, but without limiting the invention, the points of intersection (I1, I2) are located within the region R2 for positioning the breast M. Maximizing the uniformity of irradiation among the different X-ray tubes 4 allows the detector device 2 to receive substantially the same quantity of energy from each X-ray tube 4 under equal conditions of released energy (or dose) from each X-ray tube 4.

According to this aspect, it should be noted that the principal directions (D1, D2, D3, D4, D5) of irradiation of the different tubes (4a, 4b, 4c, 4d, 4e), although they lie substantially in the same plane, do not intersect at one point before striking the first plane 3 of the detector device 2.

Also in the preferred embodiment, the apparatus 1 comprises a second frame 14 and the first frame 12 is rotatably supported relative to the second frame 14.

More specifically, the first frame 12 is rotatable about an axis B (preferably horizontal) parallel to the second plane 5.

It should also be noted that, in the preferred embodiment, the axis B lies in the second plane 5 and, still more preferably, passes through the centre A of the ring 28.

It should be borne in mind that advantageously rotating the ring 28 about the axis B allows mammography / tomosynthesis to be performed with the patient lying on an examination couch. According to this aspect, the patient is preferably made to lie on her back on the examination couch and the ring 28 is rotated through approximately 90 degrees about the axis B to a predetermined position under the couch.

In Figure 1, the reference numeral 15 denotes a mounting shaft for supporting the first frame 12 and the reference numeral 16 denotes means for rotationally driving the shaft 15, both forming part of the apparatus 1.

It should be noted that the mounting shaft 15 and the means 16 for rotationally driving the shaft 15 define means 17 for rotating the first frame 12 relative to the second frame 14.

It should also be noted that in the preferred embodiment, the first frame 12 is movable vertically relative to the second frame 14.

In effect, the second frame comprises a telescopic portion 18 whose length may be varied to allow the first frame 12 to be lifted / lowered.

The telescopic portion 18 and the related movement means (not illustrated) define means 20 for vertical movement of the first frame 12 relative to the second frame 14.

Figure 1 shows two different vertical positions of the first frame 12, one drawn with a dashed line and the other with a continuous line.

It should be noted that in variants not illustrated, the means 20 for vertical movement of the first frame 12 relative to the second frame 14 may comprise diverse devices or means for allowing the first frame 12 to move vertically relative to the second frame 14.

It should also be noted that in one variant, the ring 28 is supported rotatably about its own centre A (or, more generally speaking, about an axis perpendicular to the plane 5).

In effect, it should be noted that the ring 28 is rotatably coupled to an element 42 which is integral with the shaft 15. In other words, the ring 28 is rotatable about its own centre A relative to the second frame 14.

It should also be noted that the reference numeral 40 denotes means for rotating the ring 28 about the axis A and forming part of the apparatus.

The rotation means 40 are associated with the element 42.

According to this aspect, it is possible to rotate the ring 28 as one about its own centre A. This rotation causes both the tubes 4 and the detector device 2 to rotate about A.

According to this aspect, it is possible to obtain images with each of the tubes 4 with the ring 28 positioned at different angles of rotation about A. It should be noted that each angular position of the ring 28 about A corresponds to a different direction of compression of the breast M.

In short, it should be noted that the first frame 12 is supported rotatably by the second frame 14 about a first axis B and about a second axis A. The axes A and B are at right angles to each other.

According to another aspect, the apparatus 1 further comprises a first contact element 21 configured to support the patient's breast M on it, and a second breast M contact element 22 which is movable to allow compression, or squeezing, of the breast M between the first contact element 21 and the second contact element 22 itself.

The first contact element 21 and the second 22 together define means 35 for the stable positioning of the breast M interposed between the detector device 2 and the X-ray tubes 4 to allow the breast M to be stably positioned.

It should be noted that the expression "stable positioning of the breast" used herein means the breast M is held in a desired position relative to the detector device 2 while mammography / tomosynthesis is being performed.

It should also be noted that the two elements (21, 22) define a region R2 for positioning the breast (that is, a region where the breast is positioned in such a way that the X-ray beams emitted by the tubes 4 pass through it).

It should be noted that in one variant, the means 35 for the stable positioning of the breast M may be movable relative to the detector device 2 (that is, relative to the frame 12), in such a way that the breast can be compressed in different positions.

It should also be noted that more generally speaking the positioning region R2 is a spatial region in which the breast can be positioned in such a way that the X-ray beams emitted by the tubes 4 can pass through it in order to perform mammography / tomosynthesis.

According to another aspect, the apparatus 1 comprises a movable biopsy device 23 for taking a sample of tissue from the breast M.

In a preferred embodiment, the biopsy device 23 comprises a pushing unit 24 movable in space and equipped with a needle 25 designed to be inserted into the breast M in order to take a biopsy of biological material, that is, organic tissue, from the breast M.

According to this aspect, before the biopsy of biological tissue is taken, at least two X-ray tubes 4 are activated individually (or one after the other) in order to detect the irradiation in the first plane 3 of the detector device 2. Preferably, but without limiting the scope of the invention, three X-ray tubes 4 are activated in any order. Still more preferably, the middle tube 4c, one of the left-hand side tubes (4a, 4b) and one of the right-hand side tubes (4c, 4d) are activated in any order.

The terms "right-hand" and "left-hand" refer to the accompanying drawings.

The processing and control unit 6 is designed to derive from the set of data detected by the detector device 2 and relating to irradiation in the first, detection plane 3, the exact position of the needle 25 relative to a defined region of the breast M under examination (this region being selectable by the operator or determinable by the unit 6 according to different criteria).

The processing and control unit 6 is also designed to determine a path to be followed by the needle 25 through the breast M in order to allow the needle 25 to take a sample of biological tissue from the region of the breast M under examination.

It should be noted that, more generally speaking, the apparatus 1 might also be configured to determine a path to be followed by the needle 25 through the breast M with two images only (one relating to the middle tube 4c and another relating to one of the side tubes).

The image relating to the middle tube 4c makes it possible to identify the positioning of the needle 25 in the plane 3 of the detector device 2, whilst the image relating to one of the side tubes in combination with the image relating to the middle tube 4c makes it possible to identify by triangulation the position of the needle 25 relative to the region under examination.

From the above description, it may be inferred that the apparatus 1 according to the invention makes it possible to identify with extreme precision, reliability and speed the trajectory of the movable biopsy device 23 for taking a sample of tissue from the patient's breast M.

In effect, the possibility of sequentially activating the stationary tubes 4 makes it possible to obtain extremely quickly the position of the needle 25 relative to the region of the breast M under examination and the path followed by the selfsame needle 25 through the breast M, no waiting time being necessary between the deactivation of one tube 4 (after detection of its irradiation in the first plane 3 of the detector device 2) and the activation of another tube 4, as is the case with prior art machines on account of the need to stabilize the position of the single X-ray source relative to the detector device.

This advantageously reduces the time needed to take the biopsy of breast tissue, thus reducing patient stress due to prolonged compression and waiting for invasive action on her body and also guarantees an accurate and precise biopsy at the selected region. Described hereinafter is the operation of the apparatus 1 according to the invention with reference to performance of tomosynthesis and mammography.

This description shall not be construed as limiting the invention, since the apparatus 1 may be used according to different methods.

The breast M is placed in the positioning region R2.

It should be noted that the breast M is compressed between the first contact element 21 and the second contact element 22 before mammography or tomosynthesis is performed.

Use of the apparatus 1 to perform tomosynthesis on the breast M entails activating individually (and in any order) each of the X-ray tubes (4a, 4b, 4c, 4d, 4e) and detecting in the first, detection plane 3 the X-rays emitted by each tube (4a, 4b, 4c, 4d, 4e).

It should be noted that the radiation emitted by each tube (4a, 4b, 4c, 4d, 4e) passes through the patient's breast M.

From the data set corresponding to the X-rays emitted by the tubes 4 and detected in the first, detection plane 3 (that is, from the signal s1), the processing and control unit 6 derives the reconstruction of the tomographic planes representing the internal, in-depth structure of the breast M.

These tomographic planes are used by the operator to diagnose the presence or absence of anomalies (for example, suspect tumour growths) in the deep tissue of the breast M.

It should be noted that, advantageously, the apparatus of the invention allows the data needed to process the tomosynthesis image to be acquired in an extremely short time (that is to say, it is characterized by considerably high speed).

In effect, the tubes (4a, 4b, 4c, 4d, 4e) can be activated in any order and without having to wait for one tube 4 to be deactivated (after its irradiation in the first plane 3 has been detected) before another tube 4 is activated. In effect, it should be noted that the stationary position of the tubes 4 relative to the detector device 2 means that no transient period is necessary to stabilize the positions of the tubes (4a, 4b, 4c, 4d, 4e). Moreover, the apparatus 1 makes it possible to obtain a tomosynthesis image of high quality since the stationary position of the tubes 4 relative to the detector device 2 avoids noise or mechanical vibrations while the radiation is being detected and which might deteriorate the quality of the projections necessary for the tomosynthesis reconstruction.

The apparatus 1 also allows mammography to be performed. In this case, only one X-ray tube 4 is activated (preferably the middle tube, labelled 4c in Figure 1).

Use of the apparatus 1 to perform mammography entails activating one X-ray tube (for example, the middle tube 4c) and detecting in the first, detection plane 3 the X-rays which are emitted by the tube 4 and which pass through the patient's breast M.

From the data set corresponding to the X-rays received in the first, detection plane 3, the processing and control unit 6 derives a radiographic image.

It should be noted that in tomosynthesis, a set of images of distinct tomographic planes of the breast M can be obtained where any lesions can be identified more easily than with mammography where the same information is superposed in a single image.

In a variant not illustrated, the arc 13 which supports the tubes 4 can rotate about the centre A of the ring 28.

In this variant, the arc 13 is rotated about the centre A of the ring 28 while keeping the position of the detector device 2 unchanged relative to the first frame 12 (that is, relative to the ring 28).

Thus, the sources 4 are fixed to an arc 13 which is movable along the circumference of the ring 28 (preferably, the arc 13 is supported by the selfsame ring 28).

This variant allows a second set of images to be obtained from source positions which are offset from the acquisition positions from which the first set of images was obtained, thereby doubling the sampling density of the angular tomosynthesis arc but still requiring an acquisition time that is significantly less than that required by a single source which must be moved to reach all the necessary positions.

Also, advantageously, when the movable biopsy device 23 is fitted to the apparatus 1, the arc 13, that is, the tubes 4, can be moved so as to facilitate mounting of the movable biopsy device 23 to the apparatus 1.

In another variant which is not illustrated, the apparatus 1 comprises means for moving the detector device 2 and configured to allow the detector to be moved relative to the tubes 4.

In light of this, it should be noted that the tubes 4 are fixed stably to the frame 12 and the detector device 2 is movable relative to the frame 12. Preferably, the means for moving the detector device 2 are configured to allow translation of the detector device 2 or a rotation about an axis (preferably about an axis parallel to A). According to yet another aspect, the apparatus 1 comprises means 30 for adjusting the spectrum of the X-ray beam F, associable with one or more of the X-ray tubes (4a, 4b, 4c, 4d, 4e), to allow adjustment of the X-ray beam F emission spectrum of the tube 4 the selfsame means 30 are associated with.

It should be noted that the term "emission spectrum" is used to mean the distribution of the X-ray beam intensity as a function of the frequencies.

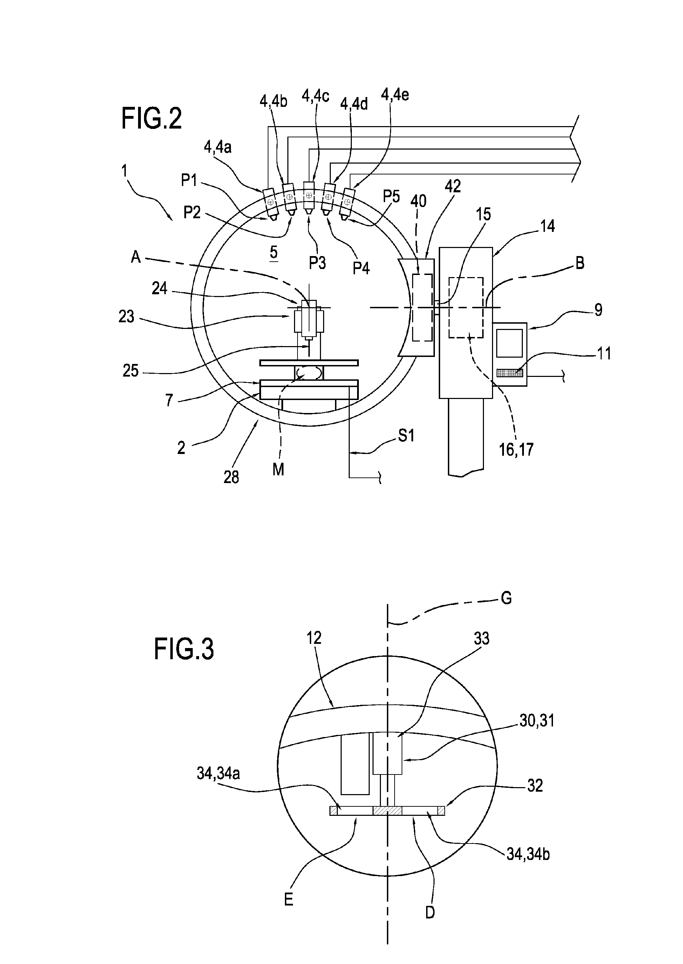

In a preferred embodiment of the means 30 for adjusting the spectrum of the X-ray beam F, illustrated in Figure 6, the means 30 comprise a device 31, associable with a corresponding X-ray tube 4, comprising a filter 34 (preferably, three filters, two of which 34a and 34b are illustrated in Figure 3), a filter support 32, and means 33 for moving the filters 34 between a non-operating position D and an operating position E of the selfsame filters.

It should be noted that each filter 34 is in the operating position E when the X-ray beam F emitted by the tube 4 the device 31 itself is associated with passes through it and, vice versa, is in the non-operating position D when the X-ray beam F does not pass through it. That way, in the operating position E and depending on its properties (geometrical and relating to the material it is made of) the filter 34 allows portions of the beam F spectrum to pass through selectively (that is to say, the beam F spectrum upstream and downstream of the filter 34 can be modified and the spectrum itself is thus adjusted).

In the embodiment of the device 31 of Figure 6, the support 32 comprises a carousel, adapted to rotate about an axis G, and two filters 34a and 34b mounted on the periphery of the carousel.

The means 33 for moving the filters 34 are configured to allow the carousel to rotate.

In this embodiment of the filtering device, the rotational drive of the carousel causes a filter 34 to move from the non-operating position D to the operating position E and vice versa.

It should be noted that, preferably, each tube 4 has a device 31 associated with it, as described above.

It should be noted that the device 31 preferably comprises three or more filters.

In another embodiment not illustrated, the means 30 for adjusting the spectrum of the beam F of one or more of the X-ray tubes 4 comprise an electronic adjustment stage configured to allow the polarization voltage of one or more of the X-ray tubes 4 to be adjusted.

In this embodiment, adjustment of the supply voltage of one or more of the X-ray tubes 4 allows adjustment of the emission spectrum of the X-ray tube 4 and/or tubes 4 which the electronic adjustment stage is associated with.

It should be noted that in any of the above described embodiments of the means 30 for adjusting the spectrum of the beam F of one or more of the X-ray tubes 4, the processing and control unit 6 is configured to control the means 30 for adjusting the emission spectrum in such a way as to adjust the emission spectrum of the tube / tubes 4 in a first and in a second configuration corresponding to the emission of a beam F with a first and a second, different spectrum, respectively.

The processing and control unit 6 is configured to derive, from the data set detected by the detector device 2 with the tube/tubes 4 in the first and in the second configuration, information regarding the nature of the internal tissue of the breast M.

In effect, it should be noted that for each X-ray tube 4, the attenuation variation of beam intensity by a tissue part with the variation of the X-ray spectrum depends on the nature of the tissue which the X-rays pass through.

It follows that the relative contrast, meaning the contrast variation in the same region between the two images (that is, between a first radiographic image derived when the tube 4 emits a beam according to the first spectrum and a second radiographic image derived when the tube 4 emits a beam according to the second spectrum), depends on the nature of the tissue of the breast M.

By way of an example, the relative contrast between the radiographic images derived in the above mentioned first and second configurations is different for a region of the breast M where there is glandular tissue compared to a region where there is a tumour growth.

This aspect therefore allows the capabilities of the apparatus 1 both in tomosynthesis and in mammography to be enhanced, while maintaining an extremely high speed.

It should also be noted that this aspect allows the capabilities of the apparatus 1 both in tomosynthesis and in mammography to be enhanced through the combined use of the spectrum adjustment means and methods for subtracting the images obtained with different spectra (in this regard, it should be noted that the processing and control unit is programmed to implement these subtraction methods).

It should be noted that what is stated above means implementing techniques known as "dual energy techniques" (such as dual energy mammography or contrast-enhanced digital mammography).

The non-linear combination of different energy images (that is images with different spectra) allows production of a hybrid image - whether of mammography or of tomographic planes, depending on the data acquisition technique used - in which the contrasts of relevant structures are maintained while those of normal structures are significantly attenuated.

"Dual energy techniques" also allow the contrast between two materials to be cancelled in order to intensify the contrast between a third material and the other two.

That means that assuming, for example, that a mammary organ is made up of three types of tissue, namely glandular, adipose and cancerous tissue, the cancellation or minimization of the contrast between healthy tissue types may facilitate detection of damaged tissue.

In connection with subtraction methods, it should be noted that the two images subtracted are acquired preferably without making the patient change position (that is, keeping the breast compressed).

Also in connection with the subtraction of planar images, it should be noted that the images are acquired from the same relative position between source and detector device 2.

It should be noted that in tomosynthesis it is in any case possible to acquire two sets of projections in different positions of the source (relative to the detector device). According to this aspect, subtraction is performed in reconstructed tomographic planes (provided the orientation of the planes relative to the patient's breast is the same).

It should also be noted that the above described embodiments of the means 30 for adjusting the spectrum of the X-ray beam F may be alternative to or combinable with each other. Indeed, a combination of them in the same apparatus 1 is possible.

According to a further aspect, the apparatus 1 comprises a grille 7 for removing diffuse radiation.

The grille 7 for removing diffuse radiation is interposed between the X-ray tubes 4 and the detector device 2, that is, it is located along the path of the X-ray beam F so as to intercept the beam F.

More specifically, while mammography or tomosynthesis is being performed or while a biopsy is being taken (that is, during use of the apparatus 1) the grille 7 for removing diffuse radiation is interposed between the patient's breast and the detector device 2.

It should be noted that the grille 7 defines a space 8 for removing diffuse radiation configured to allow only X-rays having predetermined angles (that is, primary radiation) to pass through it.

It should be noted that in this description, the radiation corresponding to the X-ray beam F emitted by one of the X-ray tubes 4 will also be referred to as "primary radiation" while the photons deviated by the X-rays passing through a portion of the human body (that is, the breast) and propagating in different directions relative to the primary radiation will be referred to as "scattering radiation" or "diffuse radiation" or "secondary radiation".

Below is a detailed description of the grille 7 for removing diffuse radiation, forming part of the apparatus 1 according to the invention.

The grille 7 for removing diffuse radiation comprises a plurality of plates 26 which are positioned to face one another, that is, which are positioned in such a way that their respective large planar faces or planes 27 are opposed to each other.

It should be noted that the term "plate" is used to mean a substantially planar element characterized by a longitudinal direction, that is a direction of principal extension, a transverse direction and a thickness.

Preferably, the plates 26 are in the form of laminas (whose thickness is in the order of hundredths of a millimetre).

It should be noted that the plates 26 together define the space 8 for removing the diffuse radiation.

The plates 26 converge towards the same region R1 proximal to one of the X-ray tubes 4 (preferably towards a small-volume region).

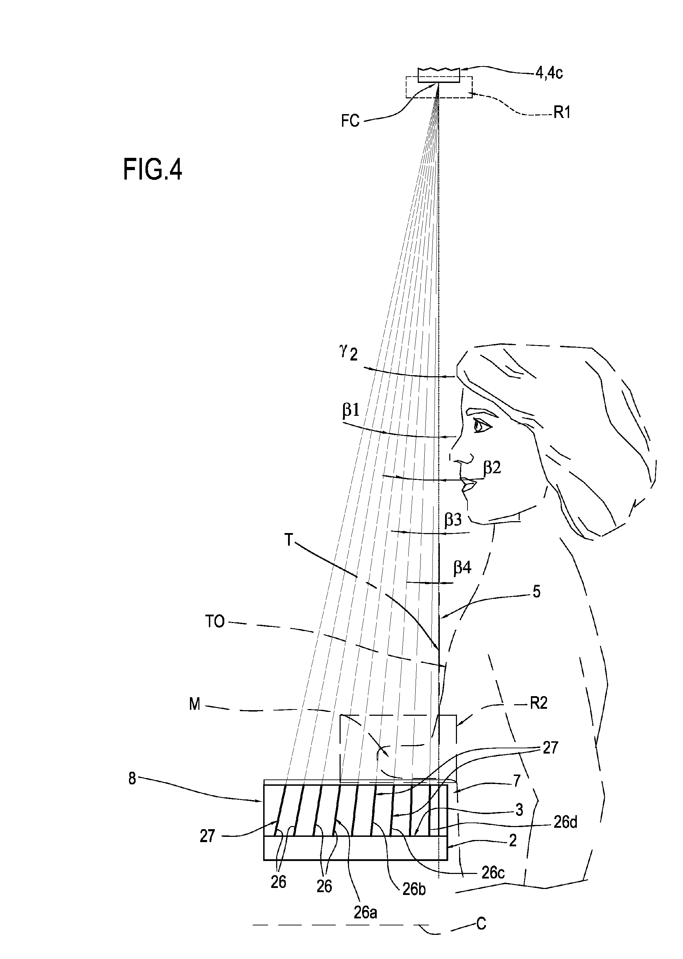

It should be noted in the side view of the apparatus 1 shown in Figure 4, the plates 26 converge towards the same point of convergence FC, that is, towards the same focus.

It should also be noted that one of the tubes 4 of the apparatus 1 is positioned at the point of convergence FC, or focus (in the embodiment illustrated, the middle tube 4c).

In other words, the plates 26 are oriented in such a way that the planes 27 defined by the large faces of the selfsame plates 26 are, in use, slightly rotated about the plane T defined by the patient's chest TO to converge at the same point of convergence or focus FC.

Preferably, the inclination of the plates 26 is a function of their distance from the second plane 5. More specifically, as may be observed in Figure 4, the inclination of the planes 27 defined by the large faces relative to the plane T defined by the patient's chest increases with distance of the plate 26 from the second plane 5 (that is, the greater the distance of the plates 26 from the second plane 5, the greater the inclination, and the smaller the distance of the plates 26 from the second plane 5, the smaller the inclination). Figure 4 schematically illustrates a portion of the grille 7 showing a plurality of plates 26. For convenience, four of the plates are labelled individually 26a, 26b, 26c, 26d, starting from the one of the four that is furthest from the second plane 5.

It should be noted that each of the plates (26a, 26b, 26c, 26d) has its own angle of inclination (β1, β2, β3, β4) relative to the plane T defined by the patient's chest TO and that these angles of inclination (β1, β2, β3, β4) decrease, starting from the plate 26a, towards the second plane 5. More specifically, in Figure 4, the planes 27 defined by the large faces of the plates (26a, 26b, 26c, 26d) converge, according to the invention, at a focus point labelled FC (in three-dimensional space, that point is a straight line).

It should be noted that the grille 7 for removing diffuse radiation is a "focused" grille (that is, the plates are arranged in such a way as to converge towards the same point of convergence or focus FC).

It is stressed that one of the tubes 4 of the apparatus 1 is positioned at the point of convergence FC, or focus (preferably, the middle tube 4c).

Thus, more generally speaking and according to the invention, the plates 26 are oriented in such a way as to converge at the point of emission of one of the X-ray tubes 4.

According to the invention, the plates 26 extend along a direction of extension K substantially parallel to a plane T defined by the patient's chest TO when the patient's breast is in the positioning region R1 (that is, when the patient is in the correct position for mammography / tomosynthesis to be performed).

It should be noted that the patient is correctly positioned when she faces the second plane 5 in front of her (in Figure 1, on the other hand, the patient is turned by 90° to the correct position, that is, she is positioned sideways relative to the second plane 5).

It should also be noted that Figure 4 schematically illustrates a breast M which is positioned correctly in the positioning region R2.

It should be noted, furthermore, that the direction of extension K is also substantially parallel to the second plane 5, that is, the plane in which the X-ray source/sources is/are movable, that is in which the X-ray source/sources is/are positioned. Moreover, in the preferred embodiment, the second plane 5 is substantially at a right angle to the first plane 3.

It should be borne in mind that the plates 26 are positioned in such a way as to substantially face towards the patient's chest TO.

It should be noted that the apparatus 1 comprises movement means (not illustrated) for the grille 7 for removing diffuse radiation, by which the grille 7 is moved at least during emission of the X-ray beam.

Preferably, these movement means are configured to allow the grille 7 for removing diffuse radiation to be translated along a direction of translation C at a right angle to the direction of extension K. It should be noted that the direction of translation C is preferably parallel to the first, detection plane 3.

Also, preferably and in the preferred embodiment illustrated in Figure 1, the direction of translation C is a direction substantially at a right angle to the second plane 5.

Preferably, the movement means are activated to move the grille 7 for removing diffuse radiation while the mammography or even tomosynthesis is being performed in such a way as to cancel the shadow produced by the plates on the detector device 2 (and hence to reduce the artificial effect it produces on the radiographic image).

According to another aspect, the grille 7 for removing diffuse radiation is characterized by a relatively high plate density along the direction C. Preferably, along the direction labelled C, the grille 7 for removing diffuse radiation comprises a linear plate density per linear centimetre that is substantially greater than half the linear pixel density of the detector device 2 along the same direction.

according to this aspect and more generally speaking, the grille 7 comprises, along the direction C, a number of plates 26 per centimetre which is substantially greater than half the number of pixels per centimetre of the detector device 2 along the same direction C.

The high plate density advantageously makes it possible to minimize the stroke of the grille 7 for removing diffuse radiation along the direction C needed to allow the shadow of the plates 26 in the first, detection plane 3 to be cancelled.

More specifically, it should be noted that a linear plate density along the direction C that is substantially greater than half the linear pixel density per linear centimetre of the detector device 2 along the direction C makes it possible to cancel the shadow caused by the presence of the grille 7 for removing diffuse radiation with a limited grille 7 stroke.

This advantageously makes it possible to minimize the field of vision loss along the direction labelled C in Figure 4.

For example, if the device has a pixel whose linear dimension is in the order of 0.1 mm and a plate density greater than 130 pairs per centimetre is chosen, the stroke of the grille 7 needed to correctly cancel the shadow on the image is less than two millimetres.

The plates 26 are made preferably of a metal with a high atomic number, such as lead, for example, or in any case of a material capable of attenuating X-rays.

The plates 26 are configured in such a way as to allow a large fraction of the primary radiation to pass through the space 8 for removing diffuse radiation and to reduce the amount of secondary radiation passing through the space 8.

The grille 7 for removing diffuse radiation therefore advantageously attenuates the secondary radiation incident upon the first, detection plane 3, and at the same time allows the primary radiation to pass through the space 8 for removing diffuse radiation.

It should be noted that, advantageously, the grille 7 for removing diffuse radiation of the apparatus 1 does not need to be removed during tomosynthesis and in fact considerably improves the quality of the radiographic image even during tomosynthesis.

More specifically, the grille 7 for removing diffuse radiation makes it possible, during both mammography and tomosynthesis, to increase the ratio between the contrast of the radiographic image detected with the grille 7 for removing diffuse radiation and without the grille 7.

An advantage of this aspect is that it allows both tomosynthesis and mammography to be performed without having to remove the grille 7.

The invention described is susceptible of industrial application and may be modified and adapted in several ways without thereby departing from the scope of the inventive concept. Moreover, all details of the invention may be substituted for technically equivalent elements.

## Claims

1. An apparatus (1) for performing tomosynthesis and mammography of a breast (M) of a patient, comprising:
- an X-ray detector device (2), designed to receive and detect X-rays in a first, detection plane (3);
- a plurality of X-ray sources (4), stably positioned in a second plane (5) which is substantially at a right angle to the first, detection plane (3) in a plurality of predetermined positions (P1, P2, P3, P4, P5) relative to the detector device (2), each of the sources (4) being individually activatable for emitting a corresponding X-ray beam (F) towards the first, detection plane (3);
- a region (R2) for positioning the breast (M);
- a processing and control unit (6), connected to the X-ray sources (4) for activating them individually and connected to the detector device (2) for receiving a signal (s1) relating to the X-rays which passed through the breast (M) and were detected by the detector device (2), the unit (6) being designed to derive from the signal (s1) at least one radiographic image representative of the internal structure of the patient's breast (M), the apparatus being **characterized in that** it comprises a first frame (12), having the shape of a ring (28) and to which the X-ray sources (4) are stably fixed, and a second frame (14), the first frame (12) being rotatably supported by the second frame (14).

2. The apparatus according to claim 1, wherein the sources (4) are positioned along an arc (13).

3. The apparatus according to claim 1 or 2, wherein each source (4a, 4b, 4c, 4d, 4e) of said sources (4) is positioned in such a way that it is angularly rotated relative to a corresponding axis (H1, H2, H3, H4, H5) substantially at a right angle to the second plane (5) in such a way as to even out the respective irradiation of the sources (4) on the first, detection plane (3).

4. The apparatus according to claim 3, wherein the axis (H1, H2, H3, H4, H5) of rotation of each source (4) is proximal to a point of emission of the beam (F) of the respective source (4).

5. The apparatus according to claim 1, **characterized in that** it comprises means (20) for vertical movement of the first frame (12) relative to the second frame (14), for allowing a vertical movement of the first frame (12).

6. The apparatus according to any of the foregoing claims, **characterized in that** the first frame (12) can rotate about a centre (A) of the ring (28).

7. The apparatus according to any of the foregoing claims, **characterized in that** it comprises a grille (7) for removing diffuse radiation, the grille (7) being interposed between the positioning region (R2) and the detector device (2) so that the X-rays which passed through the breast (M) pass through it, the grille (7) for removing diffuse radiation comprising a plurality of plates (26) which are positioned opposite the patient's chest (TO) when the breast (M) is positioned in the positioning region (R2), said plates (26) being angled in such a way that they converge towards the same region (R1) proximal to one of the X-ray sources (4).

8. The apparatus according to claim 7, **characterized in that** the grille (7) for removing diffuse radiation comprises, in a direction (C) at a right angle to the chest (TO) of the patient, a number of plates (26) per centimetre which is greater than half of a number of pixels per centimetre of the detector device (2) **in that** direction (C).

9. The apparatus according to claim 7 or 8, **characterized in that** it comprises means for moving the grille (7) which are designed to allow a movement of the grille (7) in a direction (C) of movement substantially at a right angle to a direction (K) of extension of the plates (26).

10. The apparatus according to any of the foregoing claims, **characterized in that** it comprises means (30) for adjusting the spectrum of the X-ray beam, associable with at least one of the X-ray sources (4), and also **characterized in that** the processing and control unit (6) is designed to control the adjusting means (30), allowing adjustment of the spectrum of the beam (F) emitted by said one of the sources (4) between a first emission spectrum and a second emission spectrum, and for deriving an image from the X-rays detected in the first, detection plane (3) and relating to the first and second emission spectra.

11. The apparatus according to the previous claim, wherein the adjusting means (30) comprise at least one attenuating filter (34) for the beam (F) and means (33) for moving the filter (34) between an operating position (E) in which the filter (34) acts on the beam (F) to define the first emission spectrum and a non-operating position (D) in which the filter (34) does not act on the beam (F), thus defining the second emission spectrum.
